# EUROPÄISCHE PATENTSCHRIFT

(11) **EP 1 849 420 B1**
(45) Veröffentlichungstag und Bekanntmachung des Hinweises auf die Patenterteilung: **02.12.2009**
(21) Anmeldenummer: 07105232.8
(22) Anmeldetag: 29.03.2007
(51) Int. Cl.: A61B 17/28

(54) **Chirurgischer Instrumentengriff und chirurgisches Instrument**
Surgical instrument handle and surgical instrument
Poignée d'instrument chirurgical et instrument chirurgical

(30) Priorität: 03.04.2006 DE 102006016214
(43) Veröffentlichungstag der Anmeldung: 31.10.2007
(73) Patentinhaber: Aesculap AG, 78532 Tuttlingen (DE)
(72) Erfinder: Mayenberger, Rupert, 78239, Rielasingen (DE); Weissgraf, Jens Ole, 78532, Tuttlingen (DE)
(74) Vertreter: Hoeger, Stellrecht & Partner Patentanwälte

(56) Entgegenhaltungen:
- EP-A2- 0 534 303
- DE-C1- 19 717 234
- US-A- 5 626 608

## Beschreibung

Die vorliegende Erfindung betrifft einen chirurgischen Instrumentengriff mit einem ersten und einem zweiten Griffteil, die relativ zueinander bewegbar angeordnet sind, mit einer die beiden Griffteile in verschiedenen angenäherten Sperrstellungen gegen ein Auseinanderbewegen sichernden lösbaren Sperre, welche ein erstes Rastelement und ein relativ zum ersten Rastelement beweglich gelagertes und mit diesem in jeder der Sperrstellungen in Eingriff stehendes zweites Rastelement umfasst, wobei das erste Rastelement unbeweglich am ersten Griffteil angeordnet ist und wobei das zweite Rastelement beweglich am zweiten Griffteil gelagert ist, wobei ein erstes Rückstellglied vorgesehen ist zum unter Vorspannung Halten des zweiten Rastelements in Richtung auf das erste Rastelement hin, wobei ein an einem der beiden Griffteile beweglich gelagertes erstes Betätigungselement zum Bewegen des zweiten Rastelements aus einer der Sperrstellungen in eine Lösestellung vorgesehen ist, in welcher das erste und das zweite Rastelement außer Eingriff stehen, so dass der erste und der zweite Griffteil relativ zueinander frei bewegbar sind.

Ferner betrifft die vorliegende Erfindung ein chirurgisches Instrument, mit einem Rahmen, mindestens einem am Rahmen beweglich gelagerten Werkzeug und einem chirurgischen Instrumentengriff, welcher einen ersten und einen zweiten Griffteil umfasst, die relativ zueinander bewegbar angeordnet sind, mit einer die beiden Griffteile in verschiedenen angenäherten Sperrstellungen gegen ein Auseinanderbewegen sichernden lösbaren Sperre, welche ein erstes Rastelement und ein relativ zum ersten Rastelement beweglich gelagertes und mit diesem in jeder der Sperrstellungen in Eingriff stehendes zweites Rastelement umfasst, wobei das erste Rastelement unbeweglich am ersten Griffteil angeordnet ist und wobei das zweite Rastelement beweglich am zweiten Griffteil gelagert ist, wobei ein erstes Rückstellglied vorgesehen ist zum unter Vorspannung Halten des zweiten Rastelements in Richtung auf das erste Rastelement hin, wobei ein an einem der beiden Griffteile beweglich gelagertes erstes Betätigungselement zum Bewegen des zweiten Rastelements aus einer der Sperrstellungen in eine Lösestellung vorgesehen ist, in welcher das erste und das zweite Rastelement außer Eingriff stehen, so dass der erste und der zweite Griffteil relativ zueinander frei bewegbar sind, wobei ferner ein Kraftübertragungsglied vorgesehen ist, welches einerseits mit dem mindestens einen beweglich gelagerten Werkzeugelement verbunden und andererseits mit einem der beiden Griffteile, welcher beweglich am Rahmen gelagert ist, verbunden ist zum Übertragen einer Betätigungskraft vom beweglich gelagerten Griffteil auf das mindestens eine beweglich gelagerte Werkzeugelement.

Bei minimalinvasiven chirurgischen Eingriffen werden üblicherweise endoskopische Instrumente verwendet, die einen Instrumententeil mit einem langgestreckten Instrumentenschaft sowie einen mit dem Instrumententeil verbindbaren chirurgischen Instrumentengriff der eingangs beschriebenen Art umfassen. Es sind Instrumente bekannt, bei denen der Instrumentengriff mit dem Instrumententeil lösbar verbindbar ist, bei anderen bekannten chirurgischen Instrumenten ist der Instrumentengriff mit dem Instrumententeil dauerhaft fest verbunden. Beispiele für einen Instrumentengriff und ein Instrument der eingangs beschriebenen Art sind aus der DE 197 17 234 C1 bekannt. Bei diesem Instrumentengriff kann die Sperre vorübergehend oder dauerhaft deaktiviert werden.

Nachteilig bei den bekannten Sperren ist, dass zum Lösen der Sperre, so dass die beiden Griffteile relativ zueinander frei beweglich sind, hohe Betätigungskräfte erforderlich sind, was die Handhabbarkeit und Dosierung einer Betätigungskraft für eine Bedienperson erschwert.
Es ist daher Aufgabe der vorliegenden Erfindung, einen Instrumentengriff und ein Instrument der eingangs beschriebenen Art so zu verbessern, dass eine Taktilität beim Bedienen des Instrumentengriffs für eine Bedienperson verbessert wird.

Diese Aufgabe wird bei einem Instrumentengriff der eingangs beschriebenen Art erfindungsgemäß dadurch gelöst, dass ein zweites Rückstellglied vorgesehen ist zum unter Vorspannung Halten des ersten Betätigungselements in einer Ruhestellung, wobei das erste Betätigungselement und das zweite Rastelement derart angeordnet sind und zusammenwirken, dass das erste Betätigungselement entgegen der Wirkung des zweiten Rückstellglieds von der Ruhestellung in eine Entriegelungsstellung bewegbar ist zum Überführen des zweiten Rastelements entgegen der Wirkung des ersten Rückstellglieds aus einer der Sperrstellungen in die Lösestellung.

Durch das Vorsehen eines zweiten Rückstellglieds kann eine Verrastung zwischen den beiden Rastelementen von einer Betätigung des ersten Betätigungselements vollständig getrennt werden. Dadurch können insbesondere unterschiedliche Bewegungsrichtungen für das Betätigungselement und das beweglich gelagerte zweite Rastelement vorgesehen werden. Dadurch können auch individuell gewünschte und/oder unterschiedliche Kräfte für die Betätigung des ersten Rastelements und für das unter Vorspannung Halten des zweiten Rastelements vorgegeben werden. Ein auf das erste Betätigungselement wirkender, durch das zweite Rückstellglied erzeugter Widerstand wirkt somit nicht mehr direkt auf das zweite Rastelement. Ferner können so auch Betätigungswege für das erste Betätigungselement und das zweite Rastelement nach Bedarf ausgelegt werden. Beispielsweise kann ein großer Betätigungsweg für das erste Betätigungselement vorgesehen sein, um das zweite Rastelement über nur einen kurzen Betätigungsweg aus einer der Sperrstellungen in die Lösestellung überzuführen. Dadurch wird die Taktilität des I n-strumentengriffs verbessert. Die Gefahr, dass eine Bedienperson die Sperre unabsichtlich deaktiviert, verringert sich gegenüber herkömmlichen Instrumentengriffen und Instrumenten deutlich.

Grundsätzlich wäre es denkbar, das erste Griffteil und das zweite Griffteil relativ zueinander längs einer geraden Verschiebungsbahn verschiebbar zu lagern. Vorteilhaft ist es jedoch, wenn das erste Griffteil und das zweite Griffteil relativ zueinander um eine erste Schwenkachse schwenkbar gelagert sind. Eine solche Anordnung ist einfach herzustellen. Ferner sind Bedienpersonen daran gewöhnt, Griffteile von Instrumentengriffen relativ zueinander zu verschwenken.

Ferner wäre es möglich, das zweite Rastelement am zweiten Griffteil längs einer geradlinigen Verschiebungsbahn verschiebbar zu lagern. Günstig ist es jedoch, wenn das zweite Rastelement am zweiten Griffteil um eine zweite Schwenkachse schwenkbar gelagert ist. Eine derartige Anordnung und Lagerung ist besonders einfach herzustellen, beispielsweise mittels einer einzigen Gelenkwelle.

Des Weiteren wäre es auch denkbar, das erste Betätigungselement längs einer geradlinigen Verschiebungsbahn verschiebbar zu lagern. Vorteilhafterweise ist das erste Betätigungselement jedoch um eine dritte Schwenkachse schwenkbar gelagert. Die Schwenklagerung ist besonders einfach zu realisieren. Ferner kann das erste Betätigungselement am ersten oder am zweiten Griffteil schwenkbar gelagert sein.

Um eine Bewegung des zweiten Rastelements von einer Bewegung des ersten Betätigungselements möglichst gut zu trennen, ist es günstig, wenn ein Abstand der dritten Schwenkachse von der ersten Schwenkachse kleiner ist als ein Abstand der zweiten Schwenkachse von der ersten Schwenkachse. Ferner kann so auch ein Betätigungsweg für das erste Betätigungselement auf einfache Weise größer vorgegeben werden als ein Betätigungsweg des zweiten Rastelements.

Gemäß einer bevorzugten Ausführungsform der Erfindung kann vorgesehen sein, dass das erste Betätigungselement entgegen der Wirkung des zweiten Rückstellglieds in Richtung oder im Wesentlichen in Richtung auf den anderen Griffteil hin bewegbar gelagert ist. Diese Anordnung gestattet eine äußerst ergonomische Bedienung des ersten Betätigungselements. Eine Bedienperson kann beispielsweise die beiden Griffteile mit Mittelfinger und Daumen bedienen und zum Lösen der Sperre mit dem Zeigefinger derselben Hand die Sperre lösen oder durch Loslassen des ersten Betätigungselements die Sperre wieder aktivieren.

Günstigerweise trägt das erste Betätigungselement einen ersten Mitnehmer, welcher derart angeordnet ist, dass in Folge einer Bewegung des ersten Betätigungselements von der Ruhestellung in die Entriegelungsstellung das zweite Rastelement aus einer der Sperrstellungen in die Lösestellung überführbar ist. Insbesondere kann der Mitnehmer direkt am zweiten Rastelement angreifen und ein Betätigungsmoment vom ersten Betätigungselement direkt auf das zweite Rastelement übertragen zum Lösen der Sperre.

Ein besonders einfacher Aufbau des Instrumentengriffs ergibt sich dadurch, dass das erste Betätigungselement mindestens ein freies Ende aufweist und dass der erste Mitnehmer einen an dem mindestens einen freien Ende oder im Bereich des mindestens einen freien Endes des ersten Betätigungselements angeordneten Vorsprung umfasst, an dem das zweite Rastelement beim Überführen aus einer der Sperrstellungen in die Lösestellung anliegt. Der Mitnehmer liegt beispielsweise bei dieser Ausführungsform direkt am zweiten Rastelement an und wird, da er am ersten Betätigungselement angeordnet ist, in Folge einer Bewegung des ersten Betätigungselements selbst bewegt, wodurch er das zweite Rastelement entgegen der Wirkung des ersten Rückstellglieds aus einer der Sperrstellungen in die Lösestellung überführt.

Eine optimale ergonomische Ausgestaltung des Instrumentengriffs kann insbesondere dadurch erreicht werden, dass das erste Betätigungselement im Wesentlichen in Form eines L-förm igen Hebels ausgebildet ist, welcher zwei freie Enden aufweist, und dass die dritte Schwenkachse den L-fömigen Hebel im Bereich eines freien Endes schneidet. So kann auf einfache Weise ein Kniehebel ausgebildet werden, um gezielt unterschiedliche Kräfte auf das erste Betätigungselement wirken zu lassen. Insbesondere besteht bei einem L-förmigen Hebel die Möglichkeit, die beiden Rückstellglieder so anzuordnen, dass sie jeweils in Richtung eines Schenkels des L-förmigen Hebels wirken. Dadurch kann eine vollständige Entkopplung zwischen einer Bewegung des ersten Betätigungselements und des zweiten Rastelements erreicht werden.

Günstigerweise ist ein zweites Betätigungselement vorgesehen zum Überführen des zweiten Rastelements aus einer der Sperrstellungen in die Lösestellung. Beispielsweise kann das zweite Betätigungselement dazu dienen, die Sperre dauerhaft auszuschalten, also das das zweite Rastelement dauerhaft in der Lösestellung zu halten. Das zweite Betätigungselement stellt sicher, dass die Sperre nur dann dauerhaft ausgeschaltet wird, wenn das zweite Betätigungselement hierfür entsprechend betätigt wird. Durch Betätigen des ersten Betätigungselements allein kann die Sperre nicht dauerhaft ausgeschaltet werden. Dadurch kann ein unabsichtliches dauerhaftes Lösen der Sperre, wie dies bei aus dem Stand der Technik bekannten Instrumentengriffen und Instrumenten der Fall ist, verhindert werden.

Grundsätzlich wäre es denkbar, das zweite Betätigungselement längs einer geradlinigen Bahn verschiebbar anzuordnen. Besonders einfach wird der Aufbau des Instrumentengriffs jedoch dadurch, dass das zweite Betätigungselement um eine vierte Schwenkachse schwenkbar gelagert ist.

Günstig ist es, wenn das zweite Betätigungselement im Wesentlichen in Form eines um die vierte Schwenkachse schwenkbar gelagerten Schwenkhebels ausgebildet ist. Durch diese Ausgestaltung kann in einer ersten Schwenkstellung des zweiten Betätigungselements das zweite Rastelement frei bewegt werden, insbesondere kann es mit dem ersten Rastelement in Eingriff gebracht werden, um eine der möglichen Sperrstellungen einzunehmen. Ein dauerhaftes Lösen der Sperre wird durch einfaches Verschwenken des Schwenkhebels erreicht. Dies hat zudem den Vorteil, dass eine Bedienperson sofort erkennen kann, ob die Sperre gelöst ist oder nicht.

Gemäß einer bevorzugten Ausführungsform der Erfindung kann vorgesehen sein, dass dem zweiten Betätigungselement ein zweiter Mitnehmer zugeordnet ist, welcher in Folge einer Bewegung des zweiten Betätigungselements von einer EIN-Stellung, in welcher das erste und das zweite Rastelement in Eingriff bringbar sind, in eine AUS-Stellung, in welcher das erste und das zweite Rastelement dauerhaft außer Eingriff stehen, das zweite Rastelement aus einer der Sperrstellungen in die Lösestellung überführt. Durch die Bewegung des zweiten Betätigungselements wird gleichzeitig auch der zweite Mitnehmer bewegt, so dass beispielsweise auch das zweite Rastelement an einem Teil des Mitnehmers aufgleiten und aus einer der Sperrstellungen in Lösestellung bewegt werden kann.

Besonders einfach lässt sich mit dem zweiten Mitnehmer das Rastelement von einer der Sperrstellungen in die Lösestellung überführen, wenn der zweite Mitnehmer um die vierte Schwenkachse schwenkbar gelagert ist.

Vorteilhafterweise sind das zweite Betätigungselement und der zweite Mitnehmer drehfest miteinander verbunden. Dadurch kann eine Schwenkbewegung des zweiten Betätigungselements direkt eine Schwenkbewegung des zweiten Mitnehmers bewirken, so dass dieser das zweite Rastelement aus einer der Sperrstellungen in die Lösestellung überführen kann.

Vorteilhaft ist es, wenn der Mitnehmer in Form eines um die vierte Schwenkachse verschwenkbaren Schwenkhebels ausgebildet ist, welcher in der EIN-Stellung des zweiten Betätigungselements eine Stellung einnimmt, in der er das zweite Rastelement freigibt, so dass dieses mit dem ersten Rastelement in Eingriff bringbar ist, und welcher in der AUS-Stellung des zweiten Betätigungselements eine Stellung einnimmt, in der er das zweite Rastelement entgegen der Wirkung des ersten Rückstellglieds in der Lösestellung hält. Beispielsweise kann ein von der vierten Drehachse weg weisendes und um diese verschwenkbares freies Ende des zweiten Mitnehmers in Folge einer Verschwenkbewegung des zweiten Betätigungselements an das zweite Rastelement herangeführt und mit diesem in Anlage gebracht werden, so dass in Folge einer weiteren Verschwenkung des zweiten Betätigungselements der zweite Mitnehmer das zweite Rastelement aus einer der Sperrstellungen in die Lösestellung bewegt.

Grundsätzlich wäre es denkbar, den ersten Mitnehmer und den zweiten Mitnehmer separat voneinander vorzusehen, das heißt insgesamt zwei Mitnehmer. Besonders einfach wird der Aufbau des Instrumentengriffs jedoch dadurch, dass der zweite Mitnehmer den ersten Mitnehmer bildet. Es wird daher nur ein einziger Mitnehmer benötigt, der sowohl beim vorübergehenden Lösen der Sperre mittels des ersten Betätigungselements als auch beim dauerhaften Lösen der Sperre mittels des zweiten Betätigungselements zum Einsatz kommt.

Eine Mehrzahl unterschiedlicher Sperrstellungen lässt sich auf einfache Weise dadurch erreichen, dass das erste Rastelement eine Rastzahnung und dass das zweite Rastelement mindestens einen mit der Rastzahnung in Eingriff bringbaren Rastzahn umfasst.

Eine vollständige Entkopplung zwischen einer Bewegung des zweiten Rastelements und der beiden Griffteile relativ zueinander lässt sich insbesondere dadurch erreichen, dass die Rastzahnung parallel zur einer Bewegungsbahn ausgerichtet ist, welcher der erste Griffteil und der zweite Griffteil bei einer Relativbewegung folgen. Beispielsweise kann die Rastzahnung insgesamt geradlinig ausgerichtet sein, beispielsweise in Form einer Zahnstange, wenn die beiden Griffteile relativ zueinander verschiebbar gelagert sind.

Insbesondere dann, wenn die beiden Griffteile relativ zueinander verschwenkbar gelagert sind, ist es besonders vorteilhaft, wenn die Rastzahnung einen konzentrisch zur ersten Schwenkachse verlaufenden Kreisbogenabschnitt definiert. Die beiden Griffteile können auf Grund ihrer relativen Verschwenkbarkeit längs eines Kreisbogenabschnitts bewegt werden, so dass die Rastzahnung wiederum parallel zur Bewegungsbahn verläuft, wenn sie einen Kreisbogenabschnitt definiert, welcher konzentrisch zur ersten Schwenkachse verläuft.

Ein Rastwiderstand der Sperre kann verringert werden, wenn die Rastzahnung eine Mehrzahl von Zähnen und eine große Teilung mit flachen Zahnwinkeln aufweist, und wenn die Teilung größer ist als eine maximale Höhe der Zähne der Rastzahnung.

Eine besonders sichere und einfache Verrastung der beiden Rastelemente in einer der möglichen Sperrstellungen kann insbesondere dadurch erreicht werden, dass die Rastzahnung mehrere identische und äquidistant angeordnete Zähne umfasst, dass jeder Zahn eine erste und eine zweite Zahnflanke aufweist, dass die erste Zahnflanke eine flache Zahnflanke ist, dass die zweite Zahnflanke eine steile Zahnflanke ist, die parallel oder im Wesentlichen parallel zu einer Bewegungsrichtung des zweiten Rastelements verläuft und dass die erste Zahnflanke und die zweite Zahnflanke einen Zahnflankenwinkel in einem Bereich von 50° bis 90° einschließen. Vorzugsweise liegt der Zahnflankenwinkel in einem Bereich von 60° bis 80°. Durch den spitzen Zahnwinkel kann eine einfache und sichere Verrastung der beiden Rastelemente erreicht werden.

Um ein unbeabsichtigtes Lösen der Sperre zu verhindern und um eine sichere Verrastung der beiden Rastelement in einer der Sperrstellungen sicherzustellen, ist es günstig, wenn die Zähne der Rastzahnung hinterschnitten sind.

Günstigerweise weisen die beiden Rückstellglieder unterschiedliche Richtgrößen oder Härten auf. Dadurch kann beispielsweise erreicht werden, dass zum Lösen der Sperre mittels des ersten Betätigungselements größere Kräfte aufgebracht werden müssen als zum Bewegen des zweiten Rastelements aus einer der Sperrstellungen in die Lösestellung erforderlich sind. So kann die Taktilität des Instrumentengriffs deutlich verbessert werden, und zwar dadurch, dass eine Bedienperson im Wesentlichen die vom zweiten Rückstellglied ausgeübte Rückstellkraft spürt, nicht jedoch dagegen die vom ersten Rückstellglied ausgeübte Rückstellkraft, wie dies bei bekannten Instrumentengriffen und Instrumenten der Fall ist.

Zur Verbesserung der Taktilität des Instrumentengriffs ist es günstig, wenn eine vom zweiten Rückstellglied ausgeübte Rückstellkraft größer ist als eine vom ersten Rückstellglied ausgeübte Rückstellkraft. Durch die Entkopplung einer Lagerung des ersten Betätigungselements vom zweiten Rastelement ist es möglich, insbesondere das erste Rückstellglied so zu wählen, dass nur eine minimale Kraft zum außer Eingriff bringen des ersten Rastelements und des zweiten Rastelements aufgewandt werden muss, beispielsweise dann, wenn die beiden Griffteile entgegen der Wirkung der Sperre relativ zueinander bewegt werden und zum Beispiel ein zwischen zwei Zähne einer Zahnung eingreifender Rastzahn entgegen der Wirkung des ersten Rückstellglieds auf einem der Zähne aufgleitet, um anschließend in eine nächste Zahnlücke einzutauchen. Ferner kann zudem, um die Taktilität des Instruments noch weiter zu verbessern, das zweite Rückstellglied vorteilhafterweise so gewählt werden, dass eine besonders große Betätigungskraft auf das erste Betätigungselement ausgeübt werden muss, um die Sperre zu lösen. Durch die erfindungsgemäße Weiterbildung kann insbesondere quasi eine mechanische Übersetzung einer auf das erste Betätigungselement wirkenden großen Betätigungskraft in eine auf das zweite Rastelement wirkende kleine Betätigungskraft erreicht werden.

Ein besonders einfacher Aufbau des Instrumentengriffs kann zum Beispiel dadurch erreicht werden, dass das erste Rückstellglied und/oder das zweite Rückstellglied jeweils mindestens ein Federelement umfassen. Denkbar wäre auch die Verwendung eines beliebigen anderen elastischen Elements, beispielsweise eines elastisch komprimierbaren Kunststoffs.

Vorzugsweise weisen die beiden Rückstellglieder unterschiedliche Federkonstanten auf. So lässt sich auf einfache Weise die Taktilität des Instrumentengriffs wie oben beschrieben verbessern.

Vorteilhafterweise ist das mindestens eine Federelement in Form einer Schraubenfeder oder in Form einer Blattfeder ausgebildet. Blattfedern ermöglichen insbesondere eine besonders schlanke Bauform, Schraubenfedern haben insbesondere den Vorteil, dass sie über einen großen Dehnwegbereich eine lineare Federkonstante aufweisen.

Denkbar wäre es, das erste Betätigungselement am ersten Griffteil beweglich zu lagern. Vorzugsweise ist das erste Betätigungselement jedoch am zweiten Griffteil beweglich gelagert. Durch diese Anordnung ist es insbesondere möglich, alle beweglichen Teile der Sperre an einem Griffteil vorzusehen.

Gemäß einer bevorzugten Ausführungsform der Erfindung kann ferner vorgesehen sein, dass das erste Rückstellglied eine erste Kraftrichtung definiert, dass das zweite Rückstellglied eine zweite Kraftrichtung definiert und dass die erste Kraftrichtung und die zweite Kraftrichtung einen Winkel einschließen, der in einem Bereich von 60 ° bis 120 ° liegt. Vorteilhafterweise stehen die beiden Kraftrichtungen im Wesentlichen senkrecht aufeinander. Durch die Ausrichtung der beiden Rückstellglieder in der angegebenen Weise können das erste Betätigungselement und das zweite Rastelement nahezu unabhängig voneinander bewegt werden.

Günstig ist es, wenn der Instrumentengriff einen feststehenden Rahmenteil umfasst und wenn einer der beiden Griffteile unbeweglich am Rahmenteil gehalten ist. Es ist zwar grundsätzlich denkbar, beide Griffteile beweglich am Rahmenteil zu halten, jedoch verbessert sich die Handhabung des Instrumentengriffs ganz wesentlich, wenn einer der beiden Griffteile am Rahmenteil feststehend gehalten ist. Insbesondere dann, wenn der Instrumentengriff mit einem Instrumententeil zur Ausbildung beispielsweise eines endoskopischen Instruments verbunden ist, kann eine Bewegung von Werkzeugelementen am Instrumententeil bewirkt werden durch Bewegen des am Rahmenteil beweglich gelagerten Griffteils relativ zu dem am Rahmenteil feststehenden Griffteil.

Vorzugsweise ist der zweite Griffteil unbeweglich am Rahmenteil gehalten. Dies hat insbesondere den Vorteil, dass der zweite Griffteil somit im Prinzip auch feststehend ist, so dass das am zweiten Griffteil gelagerte erste Betätigungselement unabhängig vom ersten Griffteil und nicht in unbeabsichtigter Weise betätigt werden kann.

Die Stabilität des Instrumentengriffs wird insbesondere dadurch erhöht, dass die erste Schwenkachse durch den Rahmenteil verläuft.

Vorteilhaft ist es, wenn der Instrumentengriff eine Kupplung aufweist zum lösbaren Verbinden mit einem Instrumentenschaft, an dessen distalem Ende mindestens ein beweglich gelagertes Werkzeugelement angeordnet ist, und ein Kraftübertragungsglied umfasst, welches einerseits mit dem beweglich gelagerten Werkzeugelement verbunden und andererseits mit einem der beiden Griffteile, welcher relativ zur Kupplung beweglich gelagert ist, verbindbar ist zum Übertragen einer Betätigungskraft vom beweglich gelagerten Griffteil auf das mindestens eine beweglich gelagerte Werkzeugelement. Durch die Kupplung ist es möglich, den Instrumentengriff mit einem Instrumentenschaft eines Instrumententeils zu verbinden, um insgesamt ein chirurgisches Instrument auszubilden, beispielsweise ein chirurgisches Rohrschaftinstrument. Die Kupplung ermöglicht es zudem, den Instrumententeil zu Reinigungszwecken vom Instrumentengriff zu trennen. Ferner besteht die Möglichkeit, unterschiedliche Instrumententeile mit ein und demselben Instrumentengriff zu verbinden.

Günstigerweise ist zum Ausgleichen einer auf das mindestens eine Werkzeugelement wirkenden Betätigungskraft ein Rastwegausgleichselement vorgesehen. Das Rastwegausgleichselement stellt sicher, dass eine zwischen zwei relativ zueinander beweglichen Werkzeugelementen wirkende Haltekraft auf relaxierendes Gewebe sicher und in der gewünschten Weise aufrecht erhalten werden kann. Durch Vorsehen des Rastwegausgleichselements kann insbesondere eine sehr grobe Verzahnung der Rastzahnung gewählt werden, wobei trotzdem an einem Arbeitsende des Instrumententeils eine zwischen zwei relativ zueinander beweglich gelagerten Werkzeugelementen erzeugte Haltekraft oder Vorspannung fein dosiert werden kann.

Ein besonders einfacher Aufbau des Instrumentengriffs kann dadurch erreicht werden, dass das Rastwegausgleichselement das Kraftübertragungsglied umgibt und sich in proximaler Richtung am feststehenden Rahmenteil und in distaler Richtung an einer Schaftaufnahme der Kupplung oder an einem Instrumentenschaft abstützt.

Besonders kostengünstig und einfach im Aufbau wird ein Instrumentengriff, wenn das Rastwegausgleichselement eine Schraubenfeder ist.

Die eingangs gestellte Aufgabe wird bei einem chirurgischen Instrument der eingangs beschriebenen Art erfindungsgemäß dadurch gelöst, dass ein zweites Rückstellglied vorgesehen ist zum unter Vorspannung Halten des ersten Betätigungselements in einer Ruhestellung, wobei das erste Betätigungselement und das zweite Rastelement derart angeordnet sind und zusammenwirken, dass das erste Betätigungselement entgegen der Wirkung des zweiten Rückstellglieds von der Ruhestellung in eine Entriegelungsstellung bewegbar ist zum Überführen des zweiten Rastelement entgegen der Wirkung des ersten Rückstellglieds aus einer der Sperrstellungen in die Lösestellung.

Ein derart erfindungsgemäß weitergebildetes chirurgisches Instrument weist dieselben Vorteile auf wie ein bereits oben beschriebener erfindungsgemäß weitergebildeter Instrumentengriff. Insbesondere weist ein solches Instrument eine verbesserte Taktilität auf, denn der Instrumentengriff kann so ausgebildet sein, dass zum vorübergehenden Lösen der Sperre eine andere Kraftrichtung und eine andere Kraft aufgebracht werden kann als zum Bewegen des zweiten Rastelements aus einer der Sperrstellungen in die Lösestellung.

Günstig ist es, wenn das Instrument ein Rohrschaftinstrument ist, wenn der Rahmen einen langgestreckten Instrumentenschaft umfasst und wenn das Kraftübertragungsglied im Instrumentenschaft verschiebbar gelagert ist.

Ein solches Rohrschaftinstrument kann insbesondere für minimalinvasive chirurgische Eingriffe verwendet werden.

Des Weiteren ist es günstig, wenn der Instrumentengriff des chirurgischen Instruments einer der oben beschriebenen erfindungsgemäß weitergebildeten Instrumentengriffe ist. Dadurch lässt sich das Instrument für eine Bedienperson auf einfache und sichere Weise bedienen, insbesondere eine Sperre vorübergehend und/oder dauerhaft lösen.

Die nachfolgende Beschreibung einer bevorzugten Ausführungsform der Erfindung dient im Zusammenhang mit der Zeichnung der näheren Erläuterung. Es zeigen:
- Figur 1 :: eine perspektivische Ansicht eines endoskopischen Instruments;
- Figur 2:: eine teilweise geschnittene Seitenansicht des Instruments aus Figur 1 im Bereich des Instrumentengriffs in einer Sperrstellung der Sperre;
- Figur 3:: eine Ansicht ähnlich Figur 2 des Instrumentengriffs aus Figur 2 mit vorübergehend gelöster Sperre; und
- Figur 4:: eine Ansicht ähnlich Figur 2 des Instrumentengriffs aus Figur 2, jedoch mit dauerhaft gelöster Sperre.

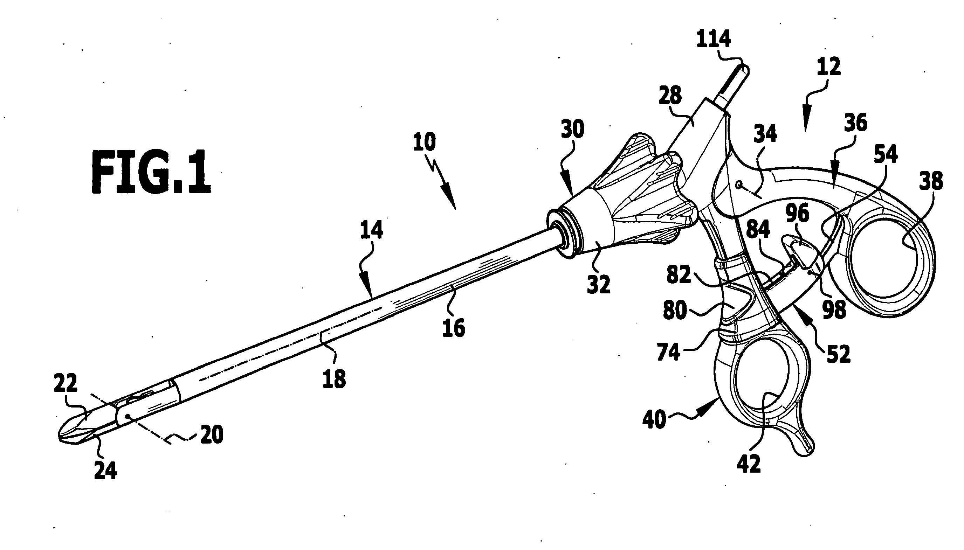

In Figur 1 ist ein insgesamt mit dem Bezugszeichen 10 versehenes chirurgisches Instrument in Form eines chirurgischen Rohrschaftinstruments dargestellt, welches einen Instrumentengriff 12 sowie einen mit dem Instrumentengriff 12 lösbar verbindbaren Instrumententeil 14 umfasst.

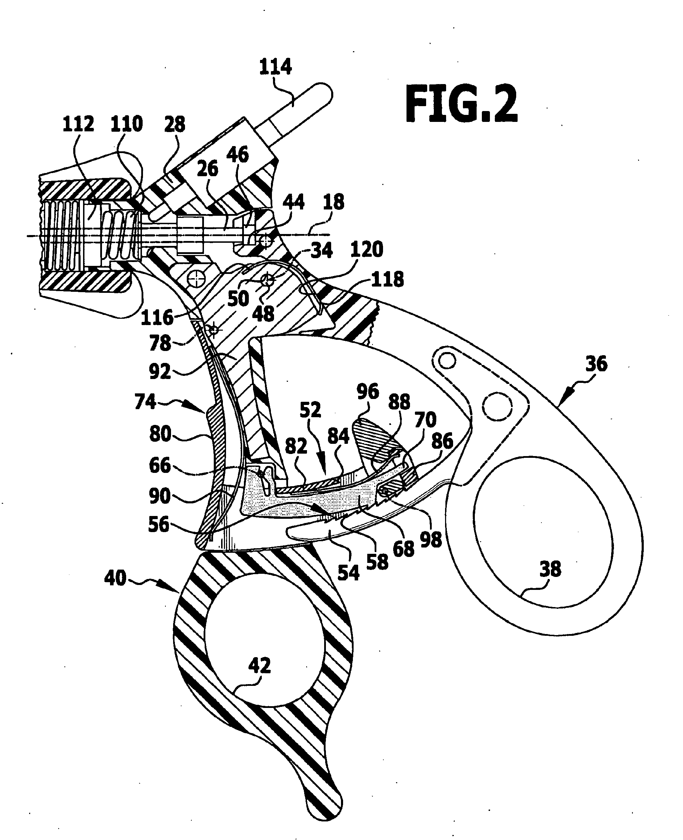

Der Instrumententeil 14 umfasst einen langgestreckten rohrförmigen Schaft 16, welcher an seinem distalen Ende um eine quer zu einer Längsachse 18 des Schafts 16 verlaufende Werkzeugschwenkachse 20 zwei verschwenkbare Werkzeugelemente 22 und 24 aufweist, die beispielsweise in Form von Schneiden zur Ausbildung einer endoskopischen Schere oder in Form von Klemmbacken zur Ausbildung einer endoskopischen Klemme ausgebildet sein können. Koaxial zur Längsachse 18 ist ein Kraftübertragungsglied in Form einer Schub-und Zugstange 26 den Schaft durchsetzend vorgesehen, deren proximales Ende in den Figuren 2 bis 4 zu erkennen ist.

Der Instrumentengriff 12 umfasst einen feststehenden Rahmenteil 28, an dem eine Kupplung 30 angeordnet ist, mit der der Instrumentengriff 12 mit dem Schaft 16 verbunden werden kann. Die Kuppplung 30 umfasst einen Drehknopf 32, welcher durch Zurückziehen in proximaler Richtung den Schaft 16 freigibt. Die Einführung des Schafts 16 in eine in Figur 2 dargestellte Schaftaufnahme 112 der Kupplung 30 führt zu einer automatischen Verrieglung des Schafts 16 am Drehknopf 32. Dadurch ist es möglich, den Drehknopf 32 und den Rahmenteil 28 relativ zueinander um die Längsachse 18 zu verdrehen. Dies gestattet es einer Bedienperson, den Instrumentengriff 12 zu halten und durch Verdrehen des Drehknopfs 32 relativ zum Rahmenteil 28 auch das distale Ende des Schafts 16 mit den Werkzeugelementen 22 und 24 in eine Drehstellung zu bewegen, in welcher beispielsweise Gewebe gehalten oder geschnitten werden soll.

Am Rahmenteil 28 ist um eine quer zur Längsachse 18 verlaufende erste Schwenkachse 34 ein erster Griffteil 36 schwenkbar gelagert. Ein freies Ende des ersten Griffteils 36 ist in Form eines Fingerrings 38 ausgebildet, in den bevorzugt ein Daumen einer Bedienperson zum Bewegen des ersten Griffteils 36 relativ zum Rahmenteil 28 eingeführt werden kann.

Ein zweiter Griffteil 40 ist distalseitig des ersten Griffteils 36 angeordnet, steht im Wesentlichen quer zur Längsachse 18 vom Rahmenteil 28 ab und ist vorzugsweise einstückig mit diesem ausgebildet. Ein freies, vom Rahmenteil 28 weg weisendes Ende des zweiten Griffteils 40 ist in Form eines Fingerrings 42 ausgebildet, durch den vorzugsweise ein Zeige-, Mittel- oder Ringfinger zum Halten des Instruments 10 eingeführt wird.

Am ersten Griffteil 36 ist jenseits der ersten Schwenkachse 34 vom Fingerring 38 aus gesehen ein freies Ende mit einer Kupplungsaufnahme 44 versehen, in welcher ein verdicktes Ende 46 des Schub- und Zugglieds 26 gehalten ist. In Folge einer Verschwenkbewegung des ersten Griffteils 36 um die Schwenkachse 34 kann so die Schub- und Zugstange 26 parallel zur Längsachse 18 in distaler und proximaler Richtung hin und her bewegt werden zum Übertragen einer Betätigungskraft vom ersten Griffteil 36 auf die Werkzeugelemente 22 und 24.

Das erste Griffteil 36 ist am Rahmenteil 28 auf einem Lagerstift 48 gelagert, der zum einen die erste Schwenkachse 34 definiert und zum anderen in einer Bohrung 50 am Rahmenteil 28 gehalten ist.

Um die Werkzeugelemente 22 und 24 in einer definierten Stellung relativ zueinander zu halten, ist eine lösbare und insgesamt mit dem Bezugszeichen 52 versehene Sperre vorgesehen, mit der die beiden Griffteile 36 und 40 in verschiedenen angenäherten Sperrstellungen gegen ein Auseinanderbewegen, wie weiter unten erläutert wird, gesichert werden können. Die Sperre 52 umfasst ein erstes Rastelement 54 in Form eines drehfest am ersten Griffteil 36 angeordneten, in Richtung auf den zweiten Griffteil 40 hin weisenden Zahnbügel. Das erste Rastelement 54 definiert einen zur ersten Schwenkachse 34 konzentrischen Kreisbogenabschnitt und ist mit einer in Richtung auf die erste Schwenkachse 34 hin gerichteten Rastzahnung 56 versehen, die eine Mehrzahl identischer und äquidistant angeordneter Zähne 58 umfasst, wobei eine erste Zahnflanke 60 der Zähne 58 im Wesentlichen in Richtung der ersten Schwenkachse 34 weist, eine zweite steile Zahnflanke 62 im Wesentlichen in Richtung auf den ersten Griffteil 36 hin. Die beiden Zahnflanken 60 und 62 schließen einen Zahnflankenwinkel von etwa 60° ein. Zudem ist die Rastzahnung 56 hinterschnitten, das heißt die Spitze der Zähne 58 steht etwas über die zweite Zahnflanke 62 in Richtung auf das erste Griffteil 36 hin vor.
Am zweiten Griffteil 40 ist um eine von einem Lagerstift 64 definierte zweite Schwenkachse 66 ein zweites Rastelement 68 verschwenkbar gelagert. Es ist im Wesentlichen L-förmig ausgebildet, mit einem kurzen, auf dem Lagerstift 64 gelagerten Schenkel und einem langgestreckten, in etwa analog dem ersten Rastelement 54 gekrümmten langen Schenkel, welcher in Richtung auf das erste Griffteil 36 hin weist und an seinem freien Ende einen flachen, in Richtung auf das erste Griffteil 36 hin weisenden Betätigungsvorsprung 70 trägt. Ferner ist am zweiten Rastelement 68 ein in Richtung auf die Rastzahnung 56 vorstehender Rastzahn 72 ausgebildet, welcher korrespondierend zu den Zähnen 58 ausgebildet ist und zwischen zwei derselben eingreifen kann. Insgesamt ist das zweite Rastelement 68 einstückig ausgebildet.

Zum Betätigen der Sperre 52 dient ein erstes Betätigungselement 74, welches um eine von einem am Rahmenteil 28 angeordneten Lagerstift 76 definierte, parallel zur ersten Schwenkachse 34 verlaufende dritte Schwenkachse 78 verschwenkbar gelagert ist.

Das erste Betätigungselement 74 ist im Wesentlichen L-förmig ausgebildet, wobei freie Schenkel des ersten Betätigungselements 74 etwa gleich lang sind. Die dritte Schwenkachse 78 durchsetzt ein in Richtung auf die Längsachse 18 hin weisendes freies Ende des ersten Betätigungselements 74. Im Übergangsbereich der beiden Schenkel des ersten Betätigungselements 74 ist in distaler Richtung weisend ein ergonomisch geformter Betätigungsbereich 80 ausgebildet.

Der andere Schenkel des ersten Betätigungselements 74 weist in Richtung auf den ersten Griffteil 36 hin und ist mit einer in Richtung auf die erste Schwenkachse 34 hin weisenden Seitenwand 82 versehen. Die Seitenwand 82 weist ein ebenfalls in Richtung auf die erste Schwenkachse 34 hin geöffnetes Fenster 84 auf. Ferner trägt das erste Betätigungselement 74 einen Anschlag 86, der an dem freien, in Richtung auf das erste Griffteil 36 hin weisenden Ende des ersten Betätigungselements 74 einem kurzen Abschnitt der Wand 82 gegenüberliegt und einen Zwischenraum freiläßt. An der Wand 82 stützt sich beiderseits des Fensters 84 eine ein erstes Rückstellglied 88 bildende Blattfeder ab, die von der ersten Schwenkachse 34 weg weisend konvex in Richtung auf das erste Rastelement 54 hin gekrümmt ist. Sie liegt zudem auf einer in Richtung auf die erste Schwenkachse 34 hin weisenden Seitenfläche des zweiten Rastelements 68 an und drückt dieses in Richtung auf das erste Rastelement 54.

Ein zweites Rückstellglied 90 in Form einer weiteren Blattfeder ist in distaler Richtung konkav gewölbt und stützt sich mit ihren beiden freien Enden auf einer Innenfläche des den Betätigungsbereich 80 tragenden Schenkels des ersten Betätigungselements 74 ab. Die konvex in proximaler Richtung gewölbte Außenfläche des zweiten Rückstellglied 90 stützt sich an einer in distaler Richtung weisenden Wandfläche eines drehfest mit dem Rahmenteil 28 verbundenen Stützglieds 92 ab.

Wird der Betätigungsbereich 80 mit einer Kraft in proximaler Richtung beaufschlagt, wird das erste Betätigungselement 74 um die dritte Schwenkachse 78 verschwenkt, und zwar entgegen einer Rückstellkraft des zweiten Rückstellglieds 90. Lässt eine Bedienperson das erste Betätigungselement 74 wieder los, zwingt das zweite Rückstellglied 90 das erste Betätigungselement 74 wieder in seine distalste Stellung zurück, die in Figur 2 dargestellt.

Benachbart des Vorsprungs 86 und etwas beabstandet von diesem ist am ersten Betätigungselement 74 ein Lagerstift 94 angeordnet, auf dem ein zweites Betätigungselement 96 verschwenkbar gelagert ist. Der Lagerstift 94 definiert eine vierte Schwenkachse 98, die parallel zu den drei anderen Schwenkachsen 34, 66 und 78 verläuft. Das zweite Betätigungselement 96 ist in Form eines Schwenkhebels ausgebildet, der teilweise durch das Fenster 84 in Richtung auf die erste Schwenkachse 34 hin weisend vorsteht. Das zweite Betätigungselement 96 ist mit einer in Richtung auf den ersten Griffteil 36 hin weisenden Durchbrechung versehen, in die der Betätigungsvorsprung 70 eintauchen kann. Ferner ist am zweiten Betätigungselement 96 ein Mitnehmer 102 angeformt, und zwar in Form eines quaderförmigen Schwenkhebels. Der Mitnehmer 102 weist im Wesentlichen in eine Richtung quer zum freien Ende des zweiten Betätigungselements 96.

In einer Grundstellung des zweiten Betätigungselements 96, der sogenannten EIN-Stellung, weist das freie Ende des zweiten Betätigungselements 96 in Richtung auf die erste Schwenkachse 34 hin, der Mitnehmer 102 folglich in Richtung auf das erste Griffteil 36. Dies hat zur Folge, dass das erste Rückstellglied 88 das zweite Rastelement 68 in Richtung auf das erste Rastelement 54 hin drückt, so dass der Rastzahn 72 zwischen zwei Zähne 58 der Rastzahnung 56 eingreift. Der Instrumentengriff 12 nimmt bei unbetätigtem ersten Betätigungselement 74 eine in Figur 2 dargestellte Sperrstellung ein. Die Sperre 52 verhindert so, dass der erste Griffteil 36 vom zweiten Griffteil 40, wie weiter unten näher erläutert wird, weg verschwenkt werden kann.

Ohne das zweite Betätigungselement 96 zu betätigen, kann durch Beaufschlagen des Betätigungsbereichs 80 mit einer Betätigungskraft in Richtung des Pfeils 104 in proximaler Richtung das erste Betätigungselement 74 etwas in Richtung auf den ersten Griffteil 36 verschwenkt werden, und zwar entgegen einer vom zweiten Rückstellglied 90 ausgeübten Rückstellkraft. Zwangsläufig wird auch das zweite Betätigungselement 96, das am ersten Betätigungselement 74 um die vierte Schwenkachse 98 verschwenkbar gelagert ist, um die dritte Schwenkachse 78 verschwenkt. Insgesamt resultiert dies in einer Bewegung des Mitnehmers 102 vom ersten Rastelement 54 weg. Wird das erste Betätigungselement 74 weit genug verschwenkt, kommt der Mitnehmer 102 in Anlage an den Betätigungsvorsprung 70 und drückt diesen ebenfalls vom ersten Rastelement 54 weg, so dass das zweite Rastelement 68 insgesamt um die zweite Schwenkachse 66 verschwenkt wird. Bereits ein kleiner Schwenkwinkel reicht aus, um den Rastzahn 72 mit der Rastzahnung 56 außer Eingriff zu bringen. Die erforderliche Kraft hierfür ist wesentlich geringer als die zur Betätigung des ersten Betätigungselements 74 erforderliche Betätigungskraft, denn das zweite Rückstellglied 90 weist eine wesentlich größere Federkonstante auf als das erste Rückstellglied 88.

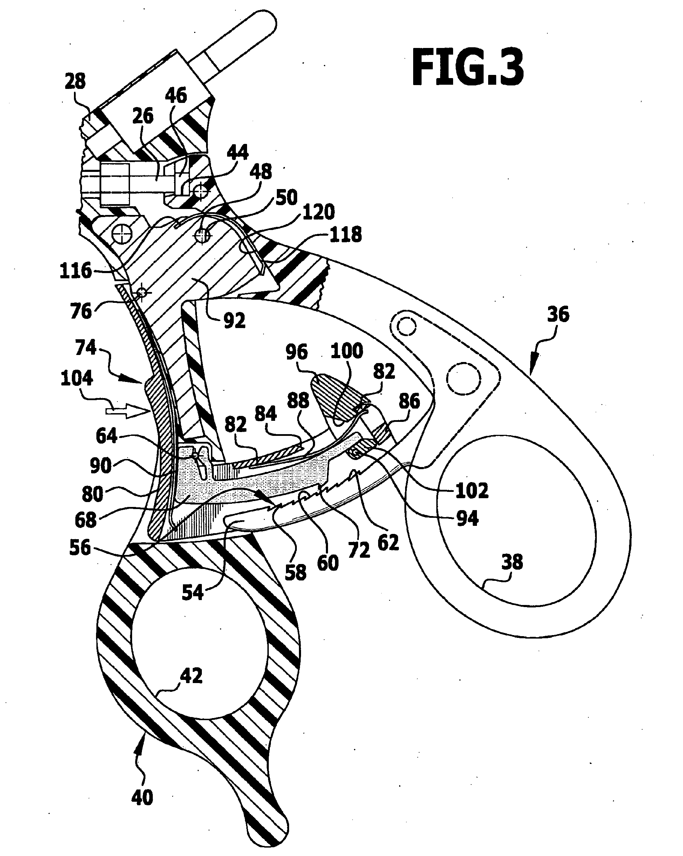

Durch das Betätigen des ersten Betätigungselements 74 wie beschrieben, wird dieses von der in Figur 2 dargestellten Ruhestellung in eine in Richtung auf den ersten Griffteil 36 hin verschwenkte Entriegelungsstellung bewegt, wobei gleichzeitig das zweite Rastelement entgegen der Wirkung des ersten Rückstellglieds aus einer der vielen möglichen Sperrstellungen in die in Figur 3 dargestellte Lösestellung bewegt wird. Wird der Betätigungsbereich 80 des ersten Betätigungselements 74 wieder frei gegeben, zwingt das erste Rückstellglied 88 das zweite Rastelement 68 wieder in Richtung auf das erste Rastelement 54 hin, das zweite Rückstellglied 90 das erste Betätigungselement 74 wieder in seine distalste Schwenkstellung.

Durch Drücken des Betätigungsbereichs 80 kann somit die Sperre 52 gelöst werden, wobei sie so nur lange gelöst bleibt, wie der Betätigungsbereich mit einer Betätigungskraft beaufschlagt wird.

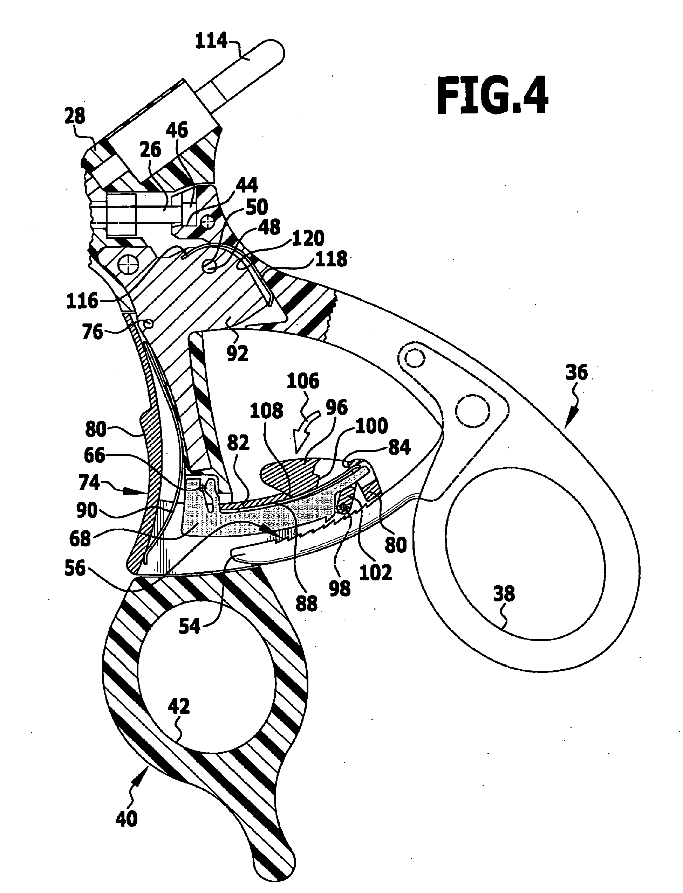

Um die Sperre 52 vollständig auszuschalten, muss das zweite Betätigungselement 96 um die vierte Schwenkachse 98 in Richtung des Pfeils 106 in Gegenuhrzeigerrichtung verschwenkt werden, bis das aus dem Fenster 84 vorstehende Ende des zweiten Betätigungselements 96 an einer das Fenster 84 in distaler Richtung begrenzenden Kante 108 der Seitenwand 82 anschlägt.
Durch diese Schwenkbewegung wird der Mitnehmer 102 ebenfalls in Gegenuhrzeigerrichtung um die vierte Schwenkachse 98 verschwenkt, so dass ein freies Ende des Mitnehmers 102, wie in Figur 4 dargestellt, den Betätigungsvorsprung 70 vom ersten Rastelement 54 weg bewegt. Es wird also durch Verschwenken des zweiten Betätigungselements 96 um die vierte Schwenkachse 98 das zweite Rastelement 68 um die zweite Schwenkachse derart verschwenkt, dass der Rastzahn 72 und die Rastzahnung 56 dauerhaft außer Eingriff gelangen. Der erste Griffteil 36 kann dann beliebig um die erste Schwenkachse relativ zum zweiten Griffteil 40 verschwenkt werden. Die Stellung des zweiten Betätigungselements 96, in der die Sperre 52 ausgeschaltet ist, wird als sogenannte AUS-Stellung bezeichnet und ist in Figur 4 dargestellt.

Zum dauerhaften Einschalten oder Aktivieren der Sperre 52 muss das zweite Betätigungselement 96 wieder aus der in Figur 4 dargestellten AUS-Stellung in Uhrzeigerrichtung um die vierte Schwenkachse 98 in die in den Figuren 2 und 3 dargestellte EI N-Stellung zurückverschwenkt werden. Das erste Rückstellglied drückt dann das zweite Rastelement 68 wieder in Richtung auf das erste Rastelement 54 hin, so dass der Rastzahn 72 wieder zwischen zwei Zähne 58 der Rastzahnung 56 eingreifen kann und ein Auseinanderbewegen der Griffteile 36 und 40 verhindert.

Aufgrund der flachen ersten Zahnflanke 60 könnte es bei zwischen den Werkzeugelementen 22 und 24 geklemmtem und relaxierendem Gewebe passieren, dass sich das Gewebe von den Werkzeugelementen löst. Um dies zu verhindern, ist eine Schraubenfeder vorgesehen, die ein Rastwegausgleichselement 110 bildet, die im proximalen Bereich der Kupplung 30 die Schub- und Zugstange 26 umgibt und sich in proximaler Richtung am feststehenden Rahmenteil 28 abstützt, in distaler Richtung an einer Schaftaufnahme 112 der Kupplung 30.

Der erfindungsgemäß weitergebildete Instrumentengriff 12 gestattet einer Bedienperson eine intuitive Bedienung. Durch Kraftbeaufschlagung des Betätigungsbereichs 80 kann die Sperre 52 gelöst werden, beim Loslassen des ersten Betätigungselements 74 ist die Sperre 52 sofort wieder aktiv. Eine dauerhafte Deaktivierung der Sperre erfolgt über ein zweites Bedienelement, nämlich das zweite Betätigungselement 96.

Der Betätigungsbereich 80 erlaubt eine ergonomische Bedienung der Sperre 52, da beispielsweise mit einem Zeigefinger die Sperre in Richtung auf den ersten Griffteil 36 hin gelöst werden kann.

Durch das im Vergleich zum zweiten Rückstellglied 90 schwächer oder weicher ausgebildete erste Rückstellglied 88 ist der Widerstand beim Verrasten der beiden Rastelemente 54 und 68 miteinander nur gering und stört die Taktilität beim Schließen der Werkzeugelemente 22 und 24 durch Verschwenken der Griffteile 36 und 40 relativ zueinander nicht. Dies folgt aus der Unabhängigkeit der Kräfte für die Bedienung der Sperre 52 und das Verrasten der Rastelemente 54 und 68. Der auf das erste Betätigungselement 74 wirkende, durch das zweite Rückstellglied 90 bewirkte Widerstand wirkt nicht auf das zweite Rastelement 68. Eine Bewegung des zweiten Rastelements 68 ist zudem unabhängig von einer Bewegung des ersten Betätigungselements 74, wenn der Rastzahn 72 mit der Rastzahnung 56 in Eingriff oder außer Eingriff gebracht wird. Ferner ist auf Grund der vorgesehenen Rastzahnung 56 mit großer Teilung und flachen ersten Zahnflanken 60 ein Rastwiderstand reduziert. Durch das Rastwegausgleichselement 110 wird eine Haltekraft der beiden Werkzeugelemente 22 und 24 definiert vorgegeben. Ferner erlaubt das Rastwegausgleichselement 110 die Aufrechterhaltung einer Haltekraft auch bei relaxierendem Gewebe. Da zudem das erste Betätigungselement 74 das zweite Rastelement 68 beidseits begrenzt, kann die Sperre 52 nicht austreten.

Das in den Figuren 1 bis 4 dargestellte Ausführungsbeispiel eines erfindungsgemäßen Instrumentengriffs ist zudem mit einem HF-Anschluss 114 versehen, so dass die Werkzeugelemente 22 und 24 über die Schub- und Zugstange 26 mit einem Hochfrequenz-(HF)-Strom beaufschlagt werden können.

Im Stützglied 92 ist in etwa zwischen der ersten Schwenkachse 34 und der Längsachse 18 ein im Wesentlichen in proximaler Richtung geöffneter Schlitz 116 angeordnet, in den ein freies Ende einer in proximaler Richtung weisend konvex gekrümmten Blattfeder 118 eingesetzt ist. Ein aus dem Schlitz 116 vorstehender Abschnitt der Blattfeder 118 liegt an einer im Wesentlichen in distaler Richtung weisenden Druckfläche 120 des ersten Griffteils 36 an, so dass durch die Blattfeder 118 der erste Griffteil 36 vom zweiten Griffteil 40 ständig weg gedrückt wird. Ist die Sperre 52 deaktiviert, so wird der erste Griffteil 36 durch die Blattfeder 118 in seine proximalste Stellung verschwenkt.

Eine Schwenkbewegung des ersten Griffteils 36 um die erste Schwenkachse 34 erfolgt somit stets gegen die Wirkung der Blattfeder 118. Deren Federkraft ist jedoch im Vergleich zur Federkraft des Rastwegausgleichselements 110 wesentlich geringer, so dass die in distaler Richtung wirkende Federkraft des Rastwegausgleichselements 110 den Schaft 16 in distaler Richtung bewegt und bei in zueinander unveränderter Stellung gehaltenen Werkzeugelementen 22 und 24 folglich auch die Schub- und Zugstange 26 in distaler Richtung gezogen wird, so dass das Rastwegausgleichselement 110, wenn der Rastzahn 72 zwischen zwei Zähne 58 der Rastzahnung 56 eingreift, die zweite Zahnflanke 62 gegen eine steile Zahnflanke des Rastzahns 72 hält und so eine Öffnungsbewegung des ersten Griffteils 36 relativ zum zweiten Griffteil 40 verhindert.

## Patentansprüche

1. Chirurgischer Instrumentengriff (12) mit einem ersten (36) und einem zweiten (40) Griffteil, die relativ zueinander bewegbar angeordnet sind, mit einer die beiden Griffteile (36, 40) in verschiedenen angenäherten Sperrstellungen gegen ein Auseinanderbewegen sichernden lösbaren Sperre (52), welche ein erstes Rastelement (54) und ein relativ zum ersten Rastelement (54) beweglich gelagertes und mit diesem in jeder der Sperrstellungen in Eingriff stehendes zweites Rastelement (68) umfasst, wobei das erste Rastelement (54) unbeweglich am ersten Griffteil (36) angeordnet ist und wobei das zweite Rastelement (68) beweglich am zweiten Griffteil (40) gelagert ist, wobei ein erstes Rückstellglied (88) vorgesehen ist zum unter Vorspannung Halten des zweiten Rastelements (68) in Richtung auf das erste Rastelement (54) hin, wobei ein an einem der beiden Griffteile (36, 40) beweglich gelagertes erstes Betätigungselement (74) zum Bewegen des zweiten Rastelements (68) aus einer der Sperrstellungen in eine Lösestellung vorgesehen ist, in welcher das erste und das zweite Rastelement (54, 68) außer Eingriff stehen, so dass der erste und der zweite Griffteil (36, 40) relativ zueinander frei bewegbar sind, **dadurch gekennzeichnet, dass** ein zweites Rückstellglied (90) vorgesehen ist zum unter Vorspannung Halten des ersten Betätigungselements (74) in einer Ruhestellung, wobei das erste Betätigungselement (74) und das zweite Rastelement (68) derart angeordnet sind und zusammenwirken, dass das erste Betätigungselement (74) entgegen der Wirkung des zweiten Rückstellglieds (90) von der Ruhestellung in eine Entriegelungsstellung bewegbar ist zum Überführen des zweiten Rastelements (68) entgegen der Wirkung des ersten Rückstellglieds (88) aus einer der Sperrstellungen in die Lösestellung.

2. Instrumentengriff nach Anspruch 1, **dadurch gekennzeichnet, dass** das erste Griffteil (36) und das zweite Griffteil (40) relativ zueinander um eine erste Schwenkachse (34) schwenkbar gelagert sind.

3. Instrumentengriff nach einem der voranstehenden Ansprüche, **dadurch gekennzeichnet, dass** das zweite Rastelement (68) am zweiten Griffteil (40) um eine zweite Schwenkachse (66) schwenkbar gelagert ist.

4. Instrumentengriff nach einem der voranstehenden Ansprüche, **dadurch gekennzeichnet, dass** das erste Betätigungselement (74) um eine dritte Schwenkachse (78) schwenkbar gelagert ist.

5. Instrumentengriff nach Anspruch 4, **dadurch gekennzeichnet, dass** ein Abstand der dritten Schwenkachse (78) von der ersten Schwenkachse (34) kleiner ist als ein Abstand der zweiten Schwenkachse (66) von der ersten Schwenkachse (34).

6. Instrumentengriff nach einem der voranstehenden Ansprüche, **dadurch gekennzeichnet, dass** das erste Betätigungselement (74) entgegen der Wirkung des zweiten Rückstellglieds (90) in Richtung oder im Wesentlichen in Richtung auf den anderen Griffteil (36) hin bewegbar gelagert ist.

7. Instrumentengriff nach einem der voranstehenden Ansprüche , **dadurch gekennzeichnet, dass** das erste Betätigungselement (74) einen ersten Mitnehmer (102) trägt, welcher derart angeordnet ist, dass in Folge einer Bewegung des ersten Betätigungselements (74) von der Ruhestellung in die Entriegelungsstellung das zweite Rastelement (68) aus einer der Sperrstellungen in die Lösestellung überführbar ist.

8. Instrumentengriff nach Anspruch 7, **dadurch gekennzeichnet, dass** das erste Betätigungselement (74) mindestens ein freies Ende aufweist und dass der erste Mitnehmer (102) einen an dem mindestens einen freien Ende oder im Bereich des mindestens einen freien Endes des ersten Betätigungselements (74) angeordneten Vorsprung (102) umfasst, an dem das zweite Rastelement (68) beim Überführen aus einer der Sperrstellungen in die Lösestellung anliegt.

9. Instrumentengriff nach Anspruch 8, **dadurch gekennzeichnet, dass** das erste Betätigungselement (74) im Wesentlichen in Form eines L-förmigen Hebels (74) ausgebildet ist, welcher zwei freie Enden aufweist, und dass die dritte Schwenkachse (78) den L-förmigen Hebel (74) im Bereich eines freien Endes schneidet.

10. Instrumentengriff nach einem der voranstehenden Ansprüche, **dadurch gekennzeichnet, dass** ein zweites Betätigungselement (96) vorgesehen ist zum Überführen des zweiten Rastelements (68) aus einer der Sperrstellungen in die Lösestellung.

11. Instrumentengriff nach Anspruch 10, **dadurch gekennzeichnet, dass** das zweite Betätigungselement (96) um eine vierte Schwenkachse (98) schwenkbar gelagert ist.

12. Instrumentengriff nach Anspruch 11, **dadurch gekennzeichnet, daß** as zweite Betätigungselement (96) im Wesentlichen in Form eines um die vierte Schwenkachse (98) schwenkbar gelagerten Schwenkhebels (96) ausgebildet ist.

13. Instrumentengriff nach einem der Ansprüche 10 bis 12, **dadurch gekennzeichnet, dass** dem zweiten Betätigungselement (96) ein zweiter Mitnehmer (102) zugeordnet ist, welcher in Folge einer Bewegung des zweiten Betätigungselements (96) von einer EIN-Stellung, in welcher das erste (54) und das zweite Rastelement (68) in Eingriff bringbar sind, in eine AUS-Stellung, in welcher das erste (54) und das zweite Rastelement (68) dauerhaft außer Eingriff stehen, das zweite Rastelement (68) aus einer der Sperrstellungen in die Lösestellung überführt.

14. Instrumentengriff nach Anspruch 13, **dadurch gekennzeichnet, dass** der zweite Mitnehmer (102) um die vierte Schwenkachse (98) schwenkbar gelagert ist.

15. Instrumentengriff nach einem der Ansprüche 13 oder 14 , **dadurch gekennzeichnet, dass** das zweite Betätigungselement (96) und der zweite Mitnehmer (102) drehfest miteinander verbunden sind.

16. Instrumentengriff nach einem der Ansprüche 13 bis 15, **dadurch gekennzeichnet, dass** der zweite Mitnehmer (102) in Form eines um die vierte (98) Schwenkachse verschwenkbaren Schwenkhebels (102) ausgebildet ist, welcher in der EIN-Stellung des zweiten Betätungselements (96) eine Stellung einnimmt, in der er das zweite Rastelement (68) freigibt, so dass dieses mit dem ersten Rastelement (54) in Eingriff bringbar ist, und welcher in der AUS-Stellung des zweiten Betätigungselements (96) eine Stellung einnimmt, in der er das zweite Rastelement (68) entgegen der Wirkung des ersten Rückstellglieds (88) in der Lösestellung hält.

17. Instrumentengriff nach einem der Ansprüche 13 bis 16, **dadurch gekennzeichnet, dass** der zweite Mitnehmer (102) den ersten Mitnehmer (102) bildet.

18. Instrumentengriff nach einem der voranstehenden Ansprüche, **dadurch gekennzeichnet, dass** das erste Rastelement (54) eine Rastzahnung (56) und dass das zweite Rastelement (68) mindestens einen mit der Rastzahnung (56) in Eingriff bringbaren Rastzahn (72) umfasst.

19. Instrumentengriff nach Anspruch 18, **dadurch gekennzeichnet, dass** die Rastzahnung (56) parallel zu einer Bewegungsbahn ausgerichtet ist, welcher der erste Griffteil (36) und der zweite Griffteil (40) bei einer Relativbewegung folgen.

20. Instrumentengriff nach einem der Ansprüche 18 oder 19, **dadurch gekennzeichnet, dass** die Rastzahnung (56) einen konzentrisch zur ersten Schwenkachse (34) verlaufenden Kreisbogenabschnitt definiert.

21. Instrumentengriff nach einem der Ansprüche 18 bis 20, **dadurch gekennzeichnet, dass** die Rastzahnung (56) eine Mehrzahl von Zähnen (58) und eine große Teilung mit flachen Zahnflankenwinkeln aufweist und dass die Teilung größer ist als eine maximale Höhe der Zähne (58) der Rastzahnung (56).

22. Instrumentengriff nach Anspruch 21, **dadurch gekennzeichnet, dass** die Rastzahnung (56) mehrere identische und äquidistant angeordnete Zähne (58) umfasst, dass jeder Zahn (58) eine erste (60) und eine zweite Zahnflanke (62) aufweist, dass die erste Zahnflanke (60) eine flache Zahnflanke (60) ist, dass die zweite Zahnflanke (62) eine steile Zahnflanke (62) ist, die parallel oder Wesentlichen parallel zu einer Bewegungsrichtung des zweiten Rastelements (68) verläuft, und dass die erste Zahnflanke (60) und die zweite Zahnflanke (62) einen Zahnflankenwinkel in einem Bereich von 50° bis 90° einschließen.

23. Instrumentengriff nach einem der Ansprüche 21 oder 22, **dadurch gekennzeichnet, dass** die Zähne (58) der Rastzahnung (56) hinterschnitten sind.

24. Instrumentengriff nach einem der voranstehenden Ansprüche, **dadurch gekennzeichnet, dass** die beiden Rückstellglieder (88, 90) unterschiedliche Richtgrößen oder Härten aufweisen.

25. Instrumentengriff nach einem der voranstehenden Ansprüche, **dadurch gekennzeichnet, dass** eine vom zweiten Rückstellglied (90) ausgeübte Rückstellkraft größer ist als eine vom ersten Rückstellglied (88) ausgeübte Rückstellkraft.

26. Instrumentengriff nach einem der voranstehenden Ansprüche, **dadurch gekennzeichnet, dass** eine vom zweiten Rückstellglied (90) in der Ruhestellung ausgeübte Rückstellkraft größer ist als eine vom ersten Rückstellglied (88) in einer der Sperrstellungen ausgeübte Rückstellkraft.

27. Instrumentengriff nach einem der voranstehenden Ansprüche, **dadurch gekennzeichnet, dass** das erste Rückstellglied (88) und/oder das zweite Rückstellglied (90) jeweils mindestens ein Federelement (88, 90) umfassen.

28. Instrumentengriff nach einem der Ansprüche 24 bis 27, **dadurch gekennzeichnet, dass** die beiden Rückstellglieder (88, 90) unterschiedliche Federkonstanten aufweisen.

29. Instrumentengriff nach einem der Ansprüche 27 oder 28, **dadurch gekennzeichnet, dass** das mindestens eine Federelement (88, 90) in Form einer Schraubenfeder oder in Form einer Blattfeder (88, 90) ausgebildet ist.

30. Instrumentengriff nach einem der voranstehenden Ansprüche, **dadurch gekennzeichnet, dass** das erste Betätigungselement (74) am zweiten Griffteil (40) beweglich gelagert ist.

31. Instrumentengriff nach einem der voranstehenden Ansprüche, **dadurch gekennzeichnet, dass** das erste Rückstellglied (88) eine erste Kraftrichtung definiert, dass das zweite Rückstellglied (90) eine zweite Kraftrichtung definiert und dass die erste Kraftrichtung und die zweite Kraftrichtung einen Winkel einschließen, der in einem Bereich von 60° bis 120° liegt.

32. Instrumentengriff nach einem der voranstehenden Ansprüche, **dadurch gekennzeichnet, dass** der Instrumentengriff (12) einen feststehenden Rahmenteil (28) umfasst und dass einer der beiden Griffteile (36, 40) unbeweglich am Rahmenteil (28) gehalten ist.

33. Instrumentengriff nach einem der voranstehenden Ansprüche, **dadurch gekennzeichnet, dass** der zweite Griffteil (40) unbeweglich am Rahmenteil (28) gehalten ist.

34. Instrumentengriff nach einem der Ansprüche 2 bis 33, **dadurch gekennzeichnet, dass** die erste Schwenkachse (34) durch den Rahmenteil (28) verläuft.

35. Instrumentengriff nach einem der voranstehenden Ansprüche, **dadurch gekennzeichnet, dass** der Instrumentengriff (12) eine Kupplung (30) aufweist zum lösbaren Verbinden mit einem Instrumentenschaft (16), an dessen distalem Ende mindestens ein beweglich gelagertes Werkzeugelement (22, 24) angeordnet ist, und ein Kraftübertragungsglied (26) umfasst, welches einerseits mit dem beweglich gelagerten Werkzeugelement (22, 24) verbunden und andererseits mit einem der beiden Griffteile (36), welcher relativ zur Kupplung (30) beweglich gelagert ist, verbindbar ist zum Übertragen einer Betätigungskraft vom beweglich gelagerten Griffteil (36) auf das mindestens eine beweglich gelagerte Werkzeugelement (22, 24).

36. Instrumentengriff nach Anspruch 35, **dadurch gekennzeichnet, dass** ein Rastwegausgleichselement (110) vorgesehen ist zum Ausgleichen einer auf das mindestens eine Werkzeugelement (22, 24) wirkenden Betätigungskraft.

37. Instrumentengriff nach Anspruch 36, **dadurch gekennzeichnet, dass** das Rastwegausgleichselement (110) das Kraftübertragungsglied (26) umgibt und sich in proximaler Richtung am feststehenden Rahmenteil (28) und in distaler Richtung an einer Schaftaufnahme (112) der Kupplung (30) oder an einem Instrumentenschaft (16) abstützt.

38. Instrumentengriff nach einem der Ansprüche 36 oder 37, **dadurch gekennzeichnet, dass** das Rastwegausgleichselement (110) eine Schraubenfeder (110) ist.

39. Chirurgisches Instrument (10), mit einem Rahmen (16, 28), mindestens einem am Rahmen (16, 28) beweglich gelagerten Werkzeug (22, 24) und einem chirurgischen Instrumentengriff (12), welcher einen ersten (36) und einen zweiten (40) Griffteil umfasst, die relativ zueinander bewegbar angeordnet sind, mit einer die beiden Griffteile (36, 40) in verschiedenen angenäherten Sperrstellungen gegen ein Auseinanderbewegen sichernden lösbaren Sperre (52), welche ein erstes Rastelement (54) und ein relativ zum ersten Rastelement (54) beweglich gelagertes und mit diesem in jeder der Sperrstellungen in Eingriff stehendes zweites Rastelement (68) umfasst, wobei das erste Rastelement (54) unbeweglich am ersten Griffteil (36) angeordnet ist und wobei das zweite Rastelement (68) beweglich am zweiten Griffteil (40) gelagert ist, wobei ein erstes Rückstellglied (88) vorgesehen ist zum unter Vorspannung Halten des zweiten Rastelements (68) in Richtung auf das erste Rastelement (54) hin, wobei ein an einem der beiden Griffteile (36, 40) beweglich gelagertes erstes Betätigungselement (74) zum Bewegen des zweiten Rastelements (68) aus einer der Sperrstellungen in eine Lösestellung vorgesehen ist, in welcher das erste und das zweite Rastelement (54, 68) außer Eingriff stehen, so dass der erste und der zweite Griffteil (36, 40) relativ zueinander frei bewegbar sind, wobei ferner ein Kraftübertragungsglied (26) vorgesehen ist, welches einerseits mit dem mindestens einen beweglich gelagerten Werkzeugelement (22, 24) verbunden und andererseits mit einem der beiden Griffteile (36), welcher beweglich am Rahmen (16, 28) gelagert ist, verbunden ist zum Übertragen einer Betätigungskraft vom beweglich gelagerten Griffteil (36) auf das mindestens eine beweglich gelagerte Werkzeugelement (22, 24), **dadurch gekennzeichnet, dass** ein zweites Rückstellglied (90) vorgesehen ist zum unter Vorspannung Halten des ersten Betätigungselements (74) in einer Ruhestellung, wobei das erste Betätigungselement (74) und das zweite Rastelement (68) derart angeordnet sind und zusammenwirken, dass das erste Betätigungselement (74) entgegen der Wirkung des zweiten Rückstellglieds (90) von der Ruhestellung in eine Entriegelungsstellung bewegbar ist zum Überführen des zweiten Rastelements (68) entgegen der Wirkung des ersten Rückstellglieds (88) aus einer der Sperrstellungen in die Lösestellung.

40. Instrument nach Anspruch 39, **dadurch gekennzeichnet, dass** das Instrument (10) ein Rohrschaftinstrument (10) ist, dass der Rahmen (16, 28) einen langgestreckten Instrumentenschaft (16) umfasst und dass das.Kraftübertragungsglied (26) im Instrumentenschaft (16) verschiebbar gelagert ist.

41. Instrument nach einem der Ansprüche 39 oder 40, **dadurch gekennzeichnet, dass** der Instrumentengriff (12) ein Instrumentengriff (12) nach einem der Ansprüche 2 bis 38 ist.

## Claims

1. Surgical instrument handle (12) with a first (36) and a second (40) handle part, which are movably arranged relative to one another, comprising a releasable locking device (52) securing the two handle parts (36, 40) in various proximate locking positions to prevent them from moving a part, which locking device comprises a first latching element (54) and a second latching element (68) which is movably mounted relative to the first latching element (54) and is engaged therewith in each of the locking positions, wherein the first latching element (54) is immovably arranged on the first handle part (36) and wherein the second latching element (68) is movably mounted on the second handle part (40), a first restoring member (88) being provided for holding the second latching element (68) with pre-loading in the direction of the first latching element (54), a first actuating element (74) movably mounted on one of the two handle parts (36, 40) being provided to move the second latching element (68) from one of the locking positions into a release position, in which the first and the second latching element (54, 68) are disengaged, with the result that the first and the second handle part (36, 40) are freely movable relative to one another, **characterised in that** a second restoring member (90) is provided to hold the first actuating element (74) with pre-loading in a rest position, the first actuating element (74) and the second latching element (68) being arranged and cooperating in such a way that the first actuating element (74) can be moved counter to the action of the second restoring member (90) from the rest position into an unlocking position to transfer the second latching element (68) counter to the action of the first restoring member (88) from one of the locking positions into the release position.

2. Instrument handle according to claim 1, **characterised in that** the first handle part (36) and the second handle part (40) are pivotably mounted relative to one another about a first pivot axis (34).

3. Instrument handle according to either of the preceding claims, **characterised in that** the second latching element (68) is pivotably mounted on the second handle part (40) about a second pivot axis (66).

4. Instrument handle according to any one of the preceding claims, **characterised in that** the first actuating element (74) is pivotably mounted about a third pivot axis (78).

5. Instrument handle according to claim 4, **characterised in that** a spacing of the third pivot axis (78) from the first pivot axis (34) is smaller than a spacing of the second pivot axis (66) from the first pivot axis (34).

6. Instrument handle according to any one of the preceding claims, **characterised in that** the first actuating element (74) is movably mounted counter to the action of the second restoring member (90) in the direction or substantially in the direction of the other handle part (36).

7. Instrument handle according to any one of the preceding claims, **characterised in that** the first actuating element (74) carries a first entrainment element (102), which is arranged in such a way that as a result of a movement of the first actuating element (74) from the rest position into the unlocking position, the second latching element (68) can be transferred from one of the locking positions into the release position.

8. Instrument handle according to claim 7, **characterised in that** the first actuating element (74) has at least one free end and **in that** the first entrainment element (102) comprises a projection (102) arranged on the at least one free end or in the region of the at least one free end of the first actuating element (74), on which projection the second latching element (68) rests when being transferred from one of the locking positions into the release position.

9. Instrument handle according to claim 8, **characterised in that** the first actuating element (74) is configured substantially in the form of an L-shaped lever (74), which has two free ends, and **in that** the third pivot axis (78) intersects the L-shaped lever (74) in the region of a free end.

10. Instrument handle according to any one of the preceding claims, **characterised in that** a second actuating element (96) is provided to transfer the second latching element (68) from one of the locking positions into the release position.

11. Instrument handle according to claim 10, **characterised in that** the second actuating element (96) is pivotably mounted about a fourth pivot axis (98).

12. Instrument handle according to claim 11, **characterised in that** the second actuating element (96) is configured substantially in the form of a pivot lever (96) pivotably mounted about the fourth pivot axis (98).

13. Instrument handle according to any one of claims 10 to 12, **characterised in that** a second entrainment element (102) is associated with the second actuating element (96) and, as a result of a movement of the second actuating element (96) from an ON position, in which the first (54) and the second latching element (68) can be brought into engagement, into an OFF position, in which the first (54) and the second latching element (68) are permanently disengaged, transfers the second latching element (68) from one of the locking positions into the release position.

14. Instrument handle according to claim 13, **characterised in that** the second entrainment element (102) is pivotably mounted about the fourth pivot axis (98).

15. Instrument handle according to either of claims 13 or 14, **characterised in that** the second actuating element (96) and the second entrainment element (102) are non-rotatably connected to one another.

16. Instrument handle according to any one of claims 13 to 15, **characterised in that** the second entrainment element (102) is configured in the form of a pivot lever (102) which can be pivoted about the fourth (98) pivot axis and which, in the ON position of the second actuating element (96) adopts a position in which it releases the second latching element (68), so that the latter can be brought into engagement with the first latching element (54), and which, in the OFF position of the second actuating element (96), adopts a position in which it holds the second latching element (68) counter to the action of the first restoring member (88) in the release position.

17. Instrument handle according to any one of claims 13 to 16, **characterised in that** the second entrainment element (102) forms the first entrainment element (102).

18. Instrument handle according to any one of the preceding claims, **characterised in that** the first latching element (54) comprises a latching tooth system (56) and **in that** the second latching element (68) comprises at least one latching tooth (72) which can be brought into engagement with the latching tooth system (56).

19. Instrument handle according to claim 18, **characterised in that** the latching tooth system (56) is oriented parallel to a movement path which is followed by the first handle part (36) and the second handle part (40) upon a relative movement.

20. Instrument handle according to either of claims 18 or 19, **characterised in that** the latching tooth system (56) defines an arc of a circle portion extending concentrically to the first pivot axis (34).

21. Instrument handle according to any one of claims 18 to 20, **characterised in that** the latching tooth system (56) has a plurality of teeth (58) and a large pitch with flat tooth flank angles and **in that** the pitch is greater than a maximum height of the teeth (58) of the latching tooth system (56).

22. Instrument handle according to claim 21, **characterised in that** the latching tooth system (56) comprises a plurality of teeth (58) which are identical and arranged equidistantly, **in that** each tooth (58) has a first (60) and a second tooth flank (62), **in that** the first tooth flank (60) is a flat tooth flank (60), **in that** the second tooth flank (62) is a steep tooth flank (62) which extends in parallel or substantially in parallel to a movement direction of the second latching element (68), and **in that** the first tooth flank (60) and a second tooth flank (62) form a tooth flank angle in a range of 50° to 90°.

23. Instrument handle according to either of claims 21 or 22, **characterised in that** the teeth (58) of the latching tooth system (56) are undercut.

24. Instrument handle according to any one of the preceding claims, **characterised in that** the two restoring members (88, 90) have different reference parameters or hardnesses.

25. Instrument handle according to any one of the preceding claims, **characterised in that** a restoring force exerted by the second restoring member (90) is greater than a restoring force exerted by the first restoring member (88).

26. Instrument handle according to any one of the preceding claims, **characterised in that** a restoring force exerted by the second restoring member (90) in the rest position is greater than a restoring force exerted by the first restoring member (88) in one of the locking positions.

27. Instrument handle according to any one of the preceding claims, **characterised in that** the first restoring member (88) and/or the second restoring member (90) in each case comprise at least one spring element (88, 90).

28. Instrument handle according to any one of claims 24 to 27, **characterised in that** the two restoring members (88, 90) have different spring constants.

29. Instrument handle according to either of claims 27 or 28, **characterised in that** the at least one spring element (88, 90) is configured in the form of a helical spring or in the form of a leaf spring (88, 90).

30. Instrument handle according to any one of the preceding claims, **characterised in that** the first actuating element (74) is movably mounted on the second handle part (40).

31. Instrument handle according to any one of the preceding claims, **characterised in that** the first restoring member (88) defines a first force direction, **in that** the second restoring member (90) defines a second force direction and **in that** the first force direction and the second force direction form an angle which is in a range of 60° to 120°.

32. Instrument handle according to any one of the preceding claims, **characterised in that** the instrument handle (12) comprises a fixed frame part (28) and **in that** one of the two handle parts (36, 40) is immovably held on the frame part (28).

33. Instrument handle according to any one of the preceding claims, **characterised in that** the second handle part (40) is immovably held on the frame part (28).

34. Instrument handle according to any one of claims 2 to 33, **characterised in that** the first pivot axis (34) extends through the frame part (28).

35. Instrument handle according to any one of the preceding claims, **characterised in that** the instrument handle (12) has a coupling (30) for releasable connection to an instrument shaft (16), on the distal end of which at least one movably mounted tool element (22, 24) is arranged, and comprises a force transmission member (26), which, on the one hand, is connected to the movably mounted tool element (22, 24) and, on the other hand, can be connected to one of the two handle parts (36), which is movably mounted relative to the coupling (30) for transmitting an actuating force from the movably mounted handle part (36) to the at least one movably mounted tool element (22, 24).

36. Instrument handle according to claim 35, **characterised in that** a latching path counterbalancing element (110) is provided for counterbalancing an actuating force acting on the at least one tool element (22, 24).

37. Instrument handle according to claim 36, **characterised in that** the latching path counterbalancing element (110) surrounds the force transmission member (26) and is supported in the proximal direction on the fixed frame part (28) and, in the distal direction, on a shaft receiver (112) of the coupling (30) or on an instrument shaft (16).

38. Instrument handle according to either of claims 36 or 37, **characterised in that** the latching path counterbalancing element (110) is a helical spring (110).

39. Surgical instrument (10) with a frame (16, 28), at least one tool (22, 24) movably mounted on the frame (16, 28), and a surgical instrument handle (12), which comprises a first (36) and a second (40) handle part, which are movably arranged relative to one another, comprising a releasable locking device (52) securing the two handle parts (36, 40) in various proximate locking positions to prevent them from moving apart, which locking device comprises a first latching element (54) and a second latching element (68) which is movably mounted relative to the first latching element (54) and is engaged therewith in each of the locking positions, wherein the first latching element (54) is immovably arranged on the first handle part (36) and wherein the second latching element (68) is movably mounted on the second handle part (40), a first restoring member (88) being provided for holding the second latching element (68) with pre-loading in the direction of the first latching element (54), a first actuating element (74) movably mounted on one of the two handle parts (36, 40) being provided to move the second latching element (68) from one of the locking positions into a release position, in which the first and the second latching element (54, 68) are disengaged, with the result that the first and the second handle part (36, 40) can be freely moved relative to one another, a force transmission element (26) furthermore being provided, which, on the one hand, is connected to the at least one movably mounted tool element (22, 24) and, on the other hand, is connected to one of the two handle parts (36), which is movably mounted on the frame (16, 28) to transmit an actuating force from the movably mounted handle part (36) to the at least one movably mounted tool element (22, 24), **characterised in that** a second restoring member (90) is provided to hold the first actuating element (74) with pre-loading in a rest position, the first actuating element (74) and the second latching element (68) being arranged and cooperating in such a way that the first actuating element (74) can be moved counter to the action of the second restoring member (90) from the rest position into an unlocking position to transfer the second latching element (68) counter to the action of the first restoring member (88) from one of the locking positions into the release position.

40. Instrument according to claim 39, **characterised in that** the instrument (10) is a tubular shaft instrument (10), **in that** the frame (16, 28) comprises an elongate instrument shaft (16) and **in that** the force transmission member (26) is displaceably mounted in the instrument shaft (16).

41. Instrument according to either of claims 39 or 40, **characterised in that** the instrument handle (12) is an instrument handle (12) according to any one of claims 2 to 38.

## Revendications

1. Poignée d'instrument chirurgical (12) comportant une première (36) et une deuxième (40) partie de poignée qui sont disposées de façon à être mobiles l'une par rapport à l'autre et un arrêt (52) déblocable sécurisant les deux parties de poignée (36, 40) dans diverses positions d'arrêt voisines contre une désolidarisation, lequel comprend un premier élément d'encliquetage (54) et un deuxième élément d'encliquetage (68) logé de façon à être mobile par rapport au premier élément d'encliquetage (54) et se trouvant en prise avec ce dernier dans chacune des positions d'arrêt, où le premier élément d'encliquetage (54) est disposé de façon à être fixé sur la première partie de poignée (36) et où le deuxième élément d'encliquetage (68) est logé de façon à être mobile sur la deuxième partie de poignée (40), où un premier élément de rappel (88) est prévu pour maintenir sous prétension le deuxième élément d'encliquetage (68) dans la direction du premier élément d'encliquetage (54), où un premier élément d'actionnement logé de façon à être mobile sur une des deux parties de poignée (36, 40) est prévu pour déplacer le deuxième élément d'encliquetage (68) d'une des positions d'arrêt à une position de déblocage dans laquelle le premier et le deuxième élément d'encliquetage (54, 68) se trouvent hors prise, de sorte que la première et la deuxième partie de poignée (36, 40) sont déplaçables librement l'une par rapport à l'autre, **caractérisée en ce qu'**un deuxième élément de rappel (90) est prévu pour maintenir sous prétension le premier élément d'actionnement (74) dans une position de repos, où le premier élément d'actionnement (74) et le deuxième élément d'encliquetage (68) sont disposés et coopèrent de telle sorte que le premier élément d'actionnement (74) peut être déplacé contre l'action du deuxième élément de rappel (90) de la position de repos à une position de déverrouillage pour amener le deuxième élément d'encliquetage (68) contre l'action du premier élément de rappel (88) d'une des positions d'arrêt à la position de déblocage.

2. Poignée d'instrument selon la revendication 1, **caractérisée en ce que** la première partie de poignée (36) et la deuxième partie de poignée (40) sont logées de façon à pouvoir pivoter l'une par rapport à l'autre autour d'un premier axe de pivotement (34).

3. Poignée d'instrument selon l'une des revendications précédentes, **caractérisée en ce que** le deuxième élément d'encliquetage (68) est logé de façon à pouvoir pivoter sur la deuxième partie de poignée (40) autour d'un deuxième axe de pivotement (66).

4. Poignée d'instrument selon l'une des revendications précédentes, **caractérisée en ce que** le premier élément d'actionnement (74) est logé de façon à pouvoir pivoter autour d'un troisième axe de pivotement (78).

5. Poignée d'instrument selon la revendication 4, **caractérisée en ce que** la distance entre le troisième axe de pivotement (78) et le premier axe de pivotement (34) est inférieure à la distance entre le deuxième axe de pivotement (66) et le premier axe de pivotement (34).

6. Poignée d'instrument selon l'une des revendications précédentes, **caractérisée en ce que** le premier élément d'actionnement (74) est logé de façon à être déplaçable contre l'action du deuxième élément de rappel (90) dans la direction ou sensiblement dans la direction de l'autre partie de poignée (36).

7. Poignée d'instrument selon l'une des revendications précédentes, **caractérisée en ce que** le premier élément d'actionnement (74) porte un premier taquet d'entraînement (102) qui est disposé de telle sorte qu'à la suite d'un déplacement du premier élément d'actionnement (74) de la position de repos à la position de déverrouillage le deuxième élément d'encliquetage (68) peut être amené d'une des positions d'arrêt à la position de déblocage.

8. Poignée d'instrument selon la revendication 7, **caractérisée en ce que** le premier élément d'actionnement (74) présente au moins une extrémité libre et le premier taquet d'entraînement (102) comprend une partie en saillie disposée sur la au moins une extrémité libre ou dans la région de la au moins une extrémité libre du premier élément d'actionnement (74), partie en saillie contre laquelle est appliqué le deuxième élément d'encliquetage (68) lors de l'amenée d'une des positions d'arrêt à la position de déblocage.

9. Poignée d'instrument selon la revendication 8, **caractérisée en ce que** le premier élément d'actionnement (74) est conçu sensiblement sous la forme d'un levier en L (74) qui présente deux extrémités libres et le troisième axe de pivotement (78) coupe le levier en L (74) au niveau d'une extrémité libre.

10. Poignée d'instrument selon l'une des revendications précédentes, **caractérisé en ce qu'**un deuxième élément d'actionnement (96) est prévu pour amener le deuxième élément d'encliquetage (68) d'une des positions d'arrêt à la position de déblocage.

11. Poignée d'instrument selon la revendication 10, **caractérisée en ce que** le deuxième élément d'actionnement (96) est logé de façon à pouvoir pivoter autour d'un quatrième axe de pivotement (98).

12. Poignée d'instrument selon la revendication 11, **caractérisée en ce que** le deuxième élément d'actionnement (96) est conçu sensiblement sous la forme d'un levier de pivotement (96) logé de façon à pouvoir pivoter autour du quatrième axe de pivotement (98).

13. Poignée d'instrument selon une des revendications 10 à 12, **caractérisée en ce qu'**au deuxième élément d'actionnement (96) correspond un deuxième taquet d'entraînement (102) qui, à la suite d'un déplacement du deuxième élément d'actionnement (96) d'une position EN PRISE dans laquelle le premier (54) et le deuxième élément d'encliquetage (68) peuvent être amenés en prise, à une position LIBRE dans laquelle le premier (54) et le deuxième élément d'encliquetage (68) se trouvent durablement hors de prise, amène le deuxième élément d'encliquetage (68) d'une des positions d'arrêt à la position de déblocage.

14. Poignée d'instrument selon la revendication 13, **caractérisée en ce que** le deuxième taquet d'entraînement (102) est logé de façon à pouvoir pivoter autour du quatrième axe de pivotement (98).

15. Poignée d'instrument selon l'une des revendications 13 ou 14, **caractérisée en ce que** le deuxième élément d'actionnement (96) et le deuxième taquet d'entraînement (102) sont reliés entre eux de façon résistant à la rotation.

16. Poignée d'instrument selon l'une des revendications 13 à 15, **caractérisée en ce que** le deuxième taquet d'entraînement (102) est conçu sous la forme d'un levier de pivotement (102) pouvant pivoter autour du quatrième axe de pivotement (98), lequel, dans la position EN PRISE du deuxième élément d'actionnement (96) adopte une position dans laquelle il libère le deuxième élément d'encliquetage (68) de sorte que ce dernier peut être mis en prise avec le premier élément d'encliquetage (54) et qui adopte dans la position LIBRE du deuxième élément d'actionnement (96) une position dans laquelle il maintient le deuxième élément d'encliquetage (68) dans la position de déblocage contre l'action du premier élément de rappel (88).

17. Poignée d'instrument selon l'une des revendications 13 à 16, **caractérisée en ce que** le deuxième taquet d'entraînement (102) constitue le premier taquet d'entraînement (102).

18. Poignée d'instrument selon l'une des revendications précédentes, **caractérisée en ce que** le premier élément d'encliquetage (54) comprend une denture d'encliquetage (56) et le deuxième élément d'encliquetage (68) présente au moins une dent d'encliquetage pouvant être mise en prise avec la denture d'encliquetage (56).

19. Poignée d'instrument selon la revendication 18, **caractérisée en ce que** la denture d'encliquetage (56) est parallèle à une trajectoire de déplacement suivie par la première partie de poignée (36) et la deuxième partie de poignée (40) lors d'un déplacement relatif.

20. Poignée d'instrument selon l'une des revendications 18 ou 19, **caractérisée en ce que** la denture d'encliquetage (56) définit un tronçon d'arc de cercle concentrique par rapport au premier axe de pivotement (34).

21. Poignée d'instrument selon l'une des revendications 18 à 20, **caractérisée en ce que** la denture d'encliquetage (56) présente une multiplicité de dents (58) et un grand pas avec des angles de faces de dents plats et le pas est plus grand qu'une hauteur maximale des dents (58) de la denture d'encliquetage (56).

22. Poignée d'instrument selon la revendication 21, **caractérisée en ce que** la denture d'encliquetage (56) comprend plusieurs dents (58) identiques et équidistantes, chaque dent (58) présente une première (60) et une deuxième (62) face de dent, la première face de dent (60) est une face de dent plate (60), la deuxième face de dent (62) est une face de dent (62) à forte pente, parallèle ou sensiblement parallèle à une direction de déplacement du deuxième élément d'encliquetage (68) et la première face de dent (60) et la deuxième face de dent (62) incluent un angle de face de dent de l'ordre de 50° à 90°.

23. Poignée d'instrument selon l'une des revendications 21 ou 22, **caractérisée en ce que** les dents (58) de la denture d'encliquetage (56) sont contredépouillées.

24. Poignée d'instrument selon l'une des revendications précédentes, **caractérisée en ce que** les deux éléments de rappel (88, 90) présentent des grandeurs directionnelles ou des duretés différentes.

25. Poignée d'instrument selon l'une des revendications précédentes, **caractérisée en ce qu'**une force de rappel exercée par le deuxième élément de rappel (90) est supérieure à une force de rappel exercée par le premier élément de rappel (88).

26. Poignée d'instrument selon l'une des revendications précédentes, **caractérisée en ce qu'**une force de rappel exercée par le deuxième élément de rappel (90) dans la position de repos est supérieure à une force de rappel exercée par le premier élément de rappel (88) dans une des positions d'arrêt.

27. Poignée d'instrument selon l'une des revendications précédentes, **caractérisée en ce que** le premier élément de rappel (88) et/ou le deuxième élément de rappel (90) comprennent chacun au moins un élément ressort (88, 90).

28. Poignée d'instrument selon l'une des revendications 24 à 27, **caractérisée en ce que** les deux éléments ressorts (88, 90) présentent des constantes de rappel différentes.

29. Poignée d'instrument selon l'une des revendications 27 ou 28, **caractérisée en ce que** le au moins un élément ressorts (88, 90) est conçu sous la forme d'un ressort cylindrique ou sous la forme d'une lame ressort (88, 90).

30. Poignée d'instrument selon l'une des revendications précédentes, **caractérisée en ce que** le premier élément de positionnement (74) est logé de façon mobile sur la deuxième partie de poignée (40).

31. Poignée d'instrument selon l'une des revendications précédentes, **caractérisée en ce que** le premier élément de rappel (88) définit une première direction de force, le deuxième élément de rappel (90) définit une deuxième direction de force et la première direction de force et la deuxième direction de force incluent un angle qui est de l'ordre de 60° à 120°.

32. Poignée d'instrument selon l'une des revendications précédentes, **caractérisée en ce que** la poignée d'instrument (12) comprend une partie de bâti (28) fixe et une des deux parties de poignée (36, 40) est maintenue de façon fixe sur la partie de bâti (28).

33. Poignée d'instrument selon l'une des revendications précédentes, **caractérisée en ce que** la deuxième partie de poignée (40) est maintenue de façon fixe sur la partie de bâti (28).

34. Poignée d'instrument selon l'une des revendications 2 à 33, **caractérisée en ce que** le premier axe de pivotement (34) passe par la partie de bâti (28).

35. Poignée d'instrument selon l'une des revendications précédentes, **caractérisée en ce que** la poignée d'instrument (12) présente un embrayage (30) en vue du raccord détachable avec une tige d'instrument (16) à l'extrémité distale de laquelle est disposé au moins un élément d'outil (22, 24) logé de façon à être mobile et comprend un élément de transmission de force (26) qui est relié d'une part avec l'élément d'outil logé de façon à être (2x) mobile (22, 24) et d'autre part avec une des deux parties de poignée (36) qui est logée de façon à être (2x) mobile par rapport à l'embrayage (36), pour transmettre une force d'actionnement de la partie de poignée (36) logée de façon à être (2x) au moins un élément outil (22, 24) logé de façon à être (2x) mobile.

36. Poignée d'instrument selon la revendication 35, **caractérisée en ce qu'**il est prévu un élément de compensation du chemin d'encliquetage (110) pour compenser une force d'actionnement s'exerçant sur le au moins un élément d'outil (22, 24).

37. Poignée d'instrument selon la revendication 36, **caractérisée en ce que** l'élément de compensation de chemin d'encliquetage (110) entoure l'élément de transmission de force (26) et s'appuie dans la direction proximale à la partie de bâti fixe (28) et dans la direction distale à un logement de tige (112) de l'embrayage (30) ou à une tige d'instrument (16).

38. Poignée d'instrument selon l'une des revendications 36 ou 37, **caractérisée en ce que** l'élément de compensation de chemin d'encliquetage (110) est un ressort cylindrique (110).

39. Instrument chirurgical (10) comportant un bâti (16, 28), au moins un outil (22, 24) mobile sur le bâti (16, 28) et une poignée d'instrument chirurgical (12) qui comprend une première (36) et une deuxième (40) partie de poignée qui sont disposées de façon à être mobiles l'une par rapport à l'autre, un arrêt (52) déblocable sécurisant les deux parties de poignée (36, 40) dans diverses positions de blocage voisines contre une désolidarisation, lequel comprend un premier élément d'encliquetage (54) et un deuxième élément d'encliquetage (68) logé de façon à être (2x) mobile par rapport au premier élément d'encliquetage (54) et se trouvant en prise avec ce dernier dans chacune des positions d'arrêt, où le premier élément d'encliquetage (54) est disposé de façon à être fixe sur la première partie de poignée (36) et où le deuxième élément d'encliquetage (68) est logé de façon à être (2x) mobile sur la deuxième partie de poignée (40), où un premier élément de rappel (88) est prévu pour maintenir sous prétension le deuxième élément d'encliquetage (68) dans la direction du premier élément d'encliquetage (54), où un premier élément d'actionnement (74) logé de façon à être (2x) mobile sur une des deux parties de poignée (36, 40) est prévu pour déplacer le deuxième élément d'encliquetage (68) d'une des positions d'arrêt à une position de déblocage dans laquelle le premier et le deuxième élément d'encliquetage (54, 68) se trouvent hors prise, de sorte que la première et la deuxième partie de poignée (36, 40) sont déplaçables librement l'une par rapport à l'autre, où il est prévu en outre un élément de transmission de force (26) qui est d'une part relié avec le au moins un élément d'outil (22, 24) logé de façon à être (2x) mobile et d'autre part avec une des deux parties de poignée (36) qui est logée de façon à être (2x) mobile sur le bâti (16, 28) pour transmettre une force d'actionnement de la partie de poignée (36) logée de façon à être (2x) mobile au au moins un élément d'outil (22, 24) logé de façon à être mobile, **caractérisé en ce qu'**un deuxième élément de rappel (90) est prévu pour maintenir sous prétension le premier élément d'actionnement (74) dans une position de repos, où le premier élément d'actionnement (74) et le deuxième élément d'encliquetage (68) sont disposés et coopèrent de telle sorte que le premier élément d'actionnement (74) peut être déplacé contre l'action du deuxième élément de rappel (90) de la position de repos à une position de déverrouillage pour amener le deuxième élément d'encliquetage (68) contre l'action du premier élément de rappel (88) d'une des positions d'arrêt à la position de déblocage.

40. Instrument selon la revendication 39, **caractérisé en ce que** l'instrument (10) est un instrument à tige tubulaire (10), le bâti (16, 28) comprend une tige d'instrument (16) très allongée et l'élément de transmission de force (26) peut coulisser dans la tige d'instrument (16).

41. Instrument selon l'une des revendications 39 ou 40, **caractérisé en ce que** la poignée d'instrument (12) est une poignée d'instrument (12) selon une des revendications 2 à 38.
